# EUROPÄISCHE PATENTSCHRIFT

(11) **EP 1 100 389 B1**
(45) Veröffentlichungstag und Bekanntmachung des Hinweises auf die Patenterteilung: **29.05.2002**
(21) Anmeldenummer: 99930976.8
(22) Anmeldetag: 23.07.1999
(51) Int. Cl.: A61B 17/72

(54) **RETROGRADER TIBIANAGEL**
RETROGRADE TIBIAL NAIL
CLOU TIBIAL RETROGRADE

(30) Priorität: 27.07.1998 EP 98810717
(43) Veröffentlichungstag der Anmeldung: 23.05.2001
(73) Patentinhaber: Stryker Trauma - Selzach AG, 2545 Selzach (CH)
(72) Erfinder: BÜHREN, Volker, D-82418 Murnau (DE); WAHL, Thomas, CH-2543 Lengnau (CH); SUTTER, Lukas, CH-9011 St. Gallen (CH); BERNHARD, Andreas, CH-2554 Meinisberg (CH); HOFMANN, Gunther, O., D-86938 Schondorf/Ammersee (DE); GONSCHOREK, Oliver, D-82419 Murnau (DE)
(74) Vertreter: Graalfs, Edo, Dipl.-Ing. Patentanwälte Hauck, Graalfs, Wehnert, Döring, Siemons
(86) Internationale Anmeldenummer: CH9900342
(87) Internationale Veröffentlichungsnummer: WO0006039

(56) Entgegenhaltungen:
- WO-A-98/24380
- DE-A- 19 619 093
- FR-A- 2 646 078
- US-A- 5 035 697

## Beschreibung

Die retrograden Implantationen von Marknägeln in Femur und Humerus sind derzeit bereits routinemäßige Osteosynthese-Verfahren. Dagegen erschien bislang die Implantation eines Marknagels in retrograder Richtung in die Tibia aufgrund anatomischer und Implantat-technischer Überlegungen als unmöglich. Insbesondere bei Brüchen im proximalen Bereich der Tibia wurden deshalb bisher z.B. Osteosynthese-Platten verwendet

Aus FR-A-2 646 078 ist ein Knochennagel bekannt geworden, der auch als Verriegelungsnagel bezeichnet wird. Der Knochennagel, der auch als Tibianagel eingesetzt werden kann und für diesen Fall an einem Ende eine Abbiegung aufweist, hat in den Endabschnitten jeweils Querbohrungen für die Aufnahme einer Knochenschraube. Der Schaft des Knochennagels ist mit achsparallelen Nuten versehen sowie einem sich axial erstreckenden Schlitz in der Wandung des hohlen Nagels, wodurch die Elastizität des Schaftes insgesamt erhöht wird.

Der Erfindung liegt die Aufgabe zugrunde, einen Tibianagel zu schaffen, der insbesondere für die retrograde Anwendung geeignet ist.

Nachfolgend wird ein Ausführungsbeispiel der Erfindung anhand der Zeichnungen erläutert. Darin zeigt:
- Figur 1: eine Seitenansicht eines Tibianagels,
- Figur 2: eine Draufsicht auf den Nagel,
- Figuren 3 bis 7: Schnitte längs den Linien III-III bis VII-VII in Figur 1, und
- Figur 8: eine Stirnansicht in Richtung des Pfeils VIII in Figur 1.

Der intramedulläre Tibianagel 1 wird aus einem biologisch verträglichen Metall hergestellt Er dient der Knochenbruchbehandlung im proximalen Teil der Tibia und zur temporären Stabilisierung von hohen Tibia-Osteotomien. Der Nagel 1 besteht aus vier Abschnitten: einem biegesteifen proximalsten Teil 2, einem proximalen, in anterior-posteriorer Richtung biegeelastischen Teil 3, einem medialen Schaft 4 und einem distalen, aufgeweiteten Teil 5 zur Aufnahme der distalen Verriegelungsschrauben 6. Der ganze Nagel ist kanuliert ausgeführt (Bohrung 7), um die Einführung mittels eines Zieldrahtes oder Führungsdrahtes zu ermöglichen. In der Regel ist er rotationssymmetrisch ausgebildet. Die Durchmesser der einzelnen Teile 2-5 variieren, sind typischerweise für den proximalen Teil 2 etwa 9 mm, beim Schaft 4 etwa 7 mm und im distalen Teil 5 etwa 10 mm. Der Nagel 1 wird in einem Längenspektrum von etwa 200 bis 400 mm hergestellt, wobei im wesentlichen die Länge des Schaftes 4 variiert wird.

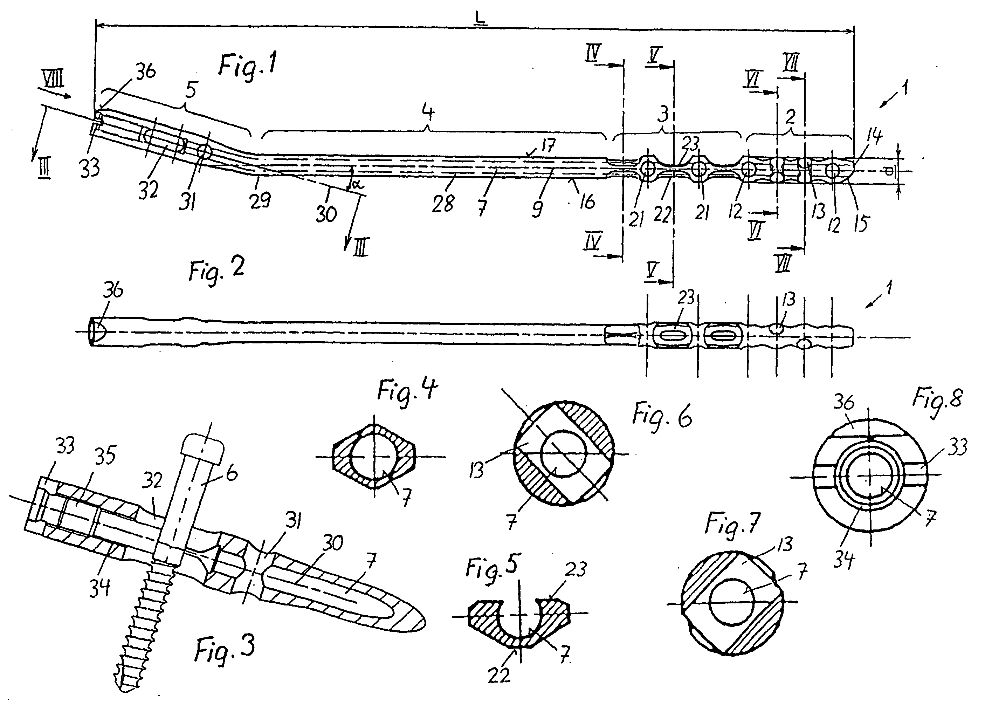

Der proximalste biegesteife Teil 2 weist mindestens zwei winkelversetzte Schraubenlöcher 12, 13 für proximale Verriegelungsschrauben auf, bei denen im Unterschied zu den Schrauben 6 nach Fig. 3 das Gewinde bis zum Schraubenkopf geschnitten ist. Mindestens ein Loch 12 liegt in medio-lateraler Richtung. Im Beispiel sind vier Löcher 12, 13 dargestellt, wobei das proximalste und das distalste in medio-lateraler Richtung liegen, und die zwei Löcher 13 dazwischen jeweils um einen Winkel um die axiale Richtung des Nagels gedreht sind, im dargestellten Beispiel um etwa 45°. Die Anordnung der Löcher 12, 13 erlaubt es, mehrere Fragmente im Bereich des Tibiaplateaus an ihrer Position zu fixieren. Im biegesteifen Teil 2 ist der Querschnitt über die ganze Länge etwa gleich. Die Biegesteifigkeit ist auf der Höhe der Löcher 12, 13 am grössten, während die Verbindungsteile zwischen den Löchern 12, 13 eine allfällige Verformung aufnehmen. Dieser Teil 2 ist um die Längsachse 9 des Nagels 1 rotationssymmetrisch ausgestaltet. Die proximale Spitze 14 hat eine kufenförmige Anfräsung 15 mit einem grossen Radius an der posterioren Seite 16, was eine erleichterte Einbringung ermöglicht. Der Radius der Anfräsung 15 ist grösser als der halbe Durchmesser d des Teils 2.

Der axial anschliessende zweite Teil 3 weist zwei Löcher 21 in medio-lateraler Richtung auf, wobei die benachbarten Abschnitte tangentiale Anfräsungen 22, 23 auf der anterioren 17 und der posterioren 16 Seite aufweisen, die dem Nagel 1 eine grössere Flexibilität in diesen Richtungen geben. Dies ist besonders wichtig, weil der Nagel 1 von der anterioren Seite 17 der Tibia eingebracht wird und dabei quasi um die Ecke gebracht werden muss. Auf der anterioren Seite 17 in diesem biegeelastischen Teil 3 sind die Anfräsungen 23 tiefer und schneiden die Bohrung 7 (Fig. 5), weil die Biegung beim Einsetzen in anteriorer Richtung ist. Die Biegeelastizität ist in anterior-posteriorer Richtung bedeutend höher als in Richtung senkrecht dazu. In einem ausgeführten Beispiel ist z.B. beim Schnitt nach Fig. 5 das Flächenträgheitsmoment um die horizontal dargestellte Achse Iₓ = 20,75 mm⁴ und senkrecht dazu I_{y} = 137,01 mm⁴. Beim Schnitt gemäss Fig. 4 sind die entsprechenden Werte Iₓ = 29,0 mm⁴ und I_{y} = 66,68 mm⁴. Das Flächenträgheitsmoment ist also in anterior-posteriorer Richtung um mindestens einen Faktor 2 kleiner als in Richtung senkrecht dazu über mindestens einen Bereich des biegeelastischen Teils 3. Im Bereich des Schnittes nach Fig. 5 ist dieser Faktor etwa 6,6, also zwischen 4 und 10. In der Gegend der Löcher 21 ist der Teil 3 kreiszylindrisch. Diese Bereiche 24 dienen als Stützen und verhindern ein Ausknicken des Teils 3 unter Druck.

Der an den biegeelastischen Teil 3 anschliessende Schaft 4 des Nagels 1 besteht aus einem Rohr 28, welches den Teil 3 und den distalen Verriegelungsteil 5 miteinander verbindet. Dieses Rohr 28 weist typischerweise einen Durchmesser zwischen 5 und 15 mm auf. Im dargestellten Beispiel wurde der Durchmesser so gewählt, dass das Rohr 28 bei genügender Festigkeit so weich wie möglich ausgestaltet wurde. Am distalen Ende dieses Rohres 28 befindet sich eine Krümmung 29, welche überleitet in den distalen Verriegelungsteil 5. Der Winkel α zwischen der Achse 9 im Rohr 28 und der Achse 30 im distalen Teil 5 kann zwischen 5° und 30° betragen. Im dargestellten Beispiel ist er 15°. Im weiteren vergrössert sich der Querschnitt des Rohrs 28 in der Krümmung 29, so dass ein stetiger Übergang vom kleineren Durchmesser des Rohres 28 zum grösseren Durchmesser des distalen Verriegelungsteils 5 stattfindet.

Dieser distale Verriegelungsteil 5 weist mindestens zwei Querbohrungen 31, 32 auf, wobei die distale Bohrung 32 als Langloch in axialer Richtung ausgeführt ist. Diese Bohrungen 31, 32 dienen der Aufnahme der distalen Verriegelungsboizen 6, die proximale Bohrung 31 für statische Verriegelungsbolzen, das Langloch 32 für dynamische oder Kompressions-Verriegelungsbolzen 6.

Am distalen Ende befindet sich eine ca. 3 bis 5 mm tiefe Nut 33, die radial durch den Durchmesser des Nagels 1 führt. Die axiale Aufbohrung des Nagels 1 ist hier am grössten. An diese Nut 33 anschliessend beginnt ein Innengewinde 34 mit geringerem Innendurchmesser in axialer proximaler Richtung, welches kurz vor der distalsten Querbohrung 32 endet. Die distale Nut 33 und das Innengewinde 34 dienen als Schnittstelle zum Zielgerät bei der Einbringung, als Andockstelle bei der Extraktion und der Aufnahme einer Kompressions- oder Verschlussschraube 35.

Die Längsbohrung des distalen Verriegelungsteils weist zudem einen grösseren Durchmesser als die Kanulation 7 des restlichen Nagels 1 auf, damit eine Kompressionsschraube 35 aufgenommen werden kann, die senkrecht auf den Bolzen 6 in der distalen Bohrung 32 drückt. Durch Eindrehen der Schraube 35 kann somit eine Kompression der Tibia erreicht werden. Am distalen, anterioren Ende des Nagels 1 befindet sich zudem eine Anfasung 36, welche Weichteilschäden vermeiden soll, falls der Nagel infolge Kompression etwas aus dem Knochen austreten sollte.

Zur Operation wird der Patient auf dem Rücken gelagert, mit Blutsperre am Oberschenkel und frei beweglich abgedecktem Bein. Der Zugang erfolgt über eine Längsinzision an der Vorderseite des distalen Unterschenkels und des oberen Sprunggelenkes mit 5 bis 7 cm Länge. Nach der Spaltung von Subcutis und Faszia werden die Retinacula der Extensoren in Längsrichtung gespalten. Der Musculus extensor halluzis longus wird nach medial retrahiert, die Tibialis anterior-Gefässe und der Nervus peronaeus profundus werden nach lateral mit einem Venenhaken retrahiert. Der anteriore Cortex der distalen Tibia wird subperiostal freigelegt. Es wird sorgfältig darauf geachtet, dass die Gelenkkapsel des oberen Sprunggelenkes nicht eröffnet wird.

Mit einem Pfriem wird der Cortex der distalen Tibia knapp über dem Ansatz der Gelenkkapsel eröffnet. Ein Führungsdraht mit leicht angebogener Spitze wird in den Markraum eingeführt und über die Fraktur bzw. die Osteotomie hinweg in die proximale Tibia bis in die Emminentia intercondylaris hochgeschoben. Der Markkanal wird mit flexiblen Bohrwellen in retrograder Richtung eröffnet. Der Aufbohrvorgang beginnt bei einem Durchmesser der Bohrköpfe von 6 mm, setzt sich in 0,5 mm Schritten fort und endet bei einem Durchmesser von 1 mm grösser als der ausgesuchte Marknagel 1.

Der Isthmus der Tibia bleibt dabei die Region für eine feste intramedulläre Fixierung des Marknagels 1. In den meisten Fällen kann der ausgewählte Marknagel 1 per Hand vorgeschoben werden. Wenn notwendig können leichte Schläge mit dem Hammer das Einsetzen des Nagels 1 erleichtern. Falls der Widerstand beim Einsetzen zu gross wird, wird ein kleinerer Nageldurchmesser gewählt. Der Nagel 1 sollte mit seinem Ende (Anfasung 36) etwas tiefer eingesetzt werden als die Oberfläche der ventralen Tibiakante. Nach Einbringen der Kompressionsschraube 35 und Ausüben der Kompression wird der Nagel 1 dann bündig mit der Knochenoberfläche abschliessen.

Die proximale Verriegelung im Tibiakopf erfolgt in Freihand-Technik durch den Tibiakopf. Dabei verwendet man vorzugsweise selbstschneidende 5 mm Schrauben. Die Verriegelungsschrauben im Tibiakopf werden vorzugsweise von medial nach lateral und von ventral nach dorsal eingesetzt. Die distalen Verriegelungsschrauben 6 werden mit Hilfe eines Zielgerätes eingesetzt.

Der beschriebene Tibianagel hat folgende Vorteile:
- Er ermöglicht ein Einsetzen in retrograder Richtung und kommt speziell bei ganz hohen Tibiakopfbrüchen zum Einsatz, wo bislang eine Marknagel-Implantation gänzlich unmöglich war.
- Als intramedullärer Kraftträger ist er früh belastbar und weitaus stabiler als herkömmliche Fixationsarten. Dies ermöglicht eine bessere physiotherapeutische Nachbehandlung des Patienten und eine rasche Genesung.
- Der Tibianagel ermöglicht eine minimal-invasive Operationstechnik und eine einfache Explantation.
- Er verbessert den Patientenkomfort.
- Die periostale Blutversorgung wird durch den Tibianagel im Markraum nicht beeinträchtigt.
- Neuronale Läsionen werden vermieden.

Der beschriebene Tibianagel eignet sich vor allem für proximale Tibiafrakturen, Umstellungs-Osteotomien, Callus-Distraktionen, Segmenttransfer, Kniegelenktransplantationen usw..

## Patentansprüche

1. Tibianagel, umfassend einen ersten Verankerungsteil (2) mit mindestens einer Querbohrung (12, 13) zur Verankerung mittels einer Verriegelungsschraube (6), einen an den ersten Verankerungsteil (2) anschließenden Schaft (4) und einen unter einem Winkel (α) an den Schaft (4) anschließenden zweiten Verankerungsteil (5), der mindestens eine weitere Querbohrung (31, 32) aufweist, **dadurch gekennzeichnet, dass** er als retrograder Tibianagel zwischen dem ersten Verankerungsteil (2) und dem Schaft (4) einen biegeelastischen Verbindungsteil (3) aufweist dessen Biegeelastizität in der die Achsen (9, 30) des Schaftes (4) und des zweiten Verankerungsteils (5) enthaltenden ersten Ebene größer ist als in einer die Achse (9) des Schaftes (4) enthaltenden, zur ersten Ebene senkrechten zweiten Ebene.

2. Tibianagel nach Anspruch 1, wobei er eine durchgehende Längsbohrung (7) aufweist.

3. Tibianagel nach Anspruch 1 oder 2, wobei er am freien Ende des ersten Verankerungsteils (2) auf der dem zweiten Verankerungsteil (5) bezüglich der Achse (7) des ersten Verankerungsteils (2) diametral gegenüberliegenden Seite eine kufenartige Anfräsung (15) aufweist.

4. Tibianagel nach einem der Ansprüche 1 bis 3, wobei der erste Verankerungsteil (2) mehrere Querbohrungen (12, 13) in unterschiedlicher Richtung aufweist, und wobei mindestens eine der Querbohrungen (12) senkrecht zur ersten Ebene verläuft.

5. Tibianagel nach einem der Ansprüche 1 bis 4, wobei der zweite Verankerungsteil (5) ein Langloch (32) aufweist sowie eine Gewinde-Längsbohrung (34) zum Einsetzen einer Kompressionsschraube (35), und wobei vorzugsweise der zweite Verankerungsteil (5) zusätzlich eine zylindrische Querbohrung (31) aufweist.

6. Tibianagel nach einem der Ansprüche 1 bis 5, wobei der Winkel (α) 5° bis 30°, vorzugsweise etwa 15° beträgt.

7. Tibianagel nach einem der Ansprüche 1 bis 6, wobei der Verbindungsteil (3) Anfräsungen (22, 23) parallel zur zweiten Ebene aufweist, und wobei vorzugsweise die Anfräsungen (23) von der einen, insbesondere der anterioren Seite her tiefer sind als von der anderen Seite und die tieferen Anfräsungen (23) die Längsbohrung (7) schneiden.

8. Tibianagel nach einem der Ansprüche 1 bis 7, wobei der Verbindungsteil (3) durch Stützkörper (24) unterteilt ist, die annähernd denselben Aussendurchmesser haben wie der erste Verar.kerungsteil (2), und wobei die Stützkörper (24) vorzugsweise weitere Querbohrungen (21) aufweisen.

9. Tibianagel nach einem der Ansprüche 1 bis 8, wobei der erste Verankerungsteil (2) zwischen den Querbohrungen (12, 13) Einschnürungen aufweist.

10. Tibianagel nach einem der Ansprüche 1 bis 9, wobei er am freien Ende des zweiten Verankerungsteils (5) einseitig eine Anfasung (36) aufweist.

11. Tibianagel nach einem der Ansprüche 1 bis 10, wobei über mindestens einen Bereich des biegeelastischen Teils (3) das Flächenträgheitsmoment um eine zur ersten Ebene senkrechten Achse um mindestens einen Faktor 2 geringer ist als um eine zur zweiten Ebene senkrechten Achse.

## Claims

1. A tibia nail, comprising a first anchoring portion (2) including at least one cross-bore (12, 13) for being anchored by means of a locking bolt (6), a shank (4) adjoining the first anchoring portion, and a second anchoring portion (5) adjoining the shank (4) under an angle (α) which has at least another cross-bore (31, 32), **characterized in that** the nail, being a retrograde tibia nail, has a flexurally resilient connection portion (3) between the first anchoring portion (2) and the shank (4), the flexural resiliency of which in the first plane containing the axes (9, 30) of the shank (4) and the second anchoring portion (5) is larger than in a second plane containing the axis (9) of the shank (4) and being perpendicular to the first plane.

2. The tibia nail according to claim 1 wherein said nail has a continuous longitudinal bore (7).

3. The tibia nail according to claim 1 or 2 wherein said nail has a skid-like milled area (15) at the free end of the first anchoring portion (2) on the side diametrically opposed to the second anchoring portion (5) with respect to the axis (7) of the first anchoring portion (2).

4. The tibia nail according to any one of claims 1 to 3 wherein the first anchoring portion (2) has several cross-bores (12, 13) in different directions and wherein at least one of the cross-bores (12) extends perpendicular to the first plane.

5. The tibia nail according to any one of claims 1 to 4 wherein the second anchoring portion (5) has an elongated hole (32) as well as a longitudinal threaded bore (34) for the insertion of a compression screw (35), and wherein the second anchoring portion (5) preferably has an additional, cylindrical cross-bore (31).

6. The tibia nail according to any one of claims 1 to 5 wherein the angle (α) ranges from 5° to 30° and preferably is about 15°.

7. The tibia nail according to any one of claims 1 to 6 wherein the connecting portion (3) has milled areas (22, 23) parallel to the second plane, and wherein the milled areas (22, 23) from one side, specifically from the anterior side, preferably are deeper than from the other side, and the deeper milled areas (23) intersect the longitudinal bore (7).

8. The tibia nail according to any one of claims 1 to 7 wherein the connecting portion (3) is subdivided by supporting bodies (24) the outside diameter of which approximately is the same than that of the first anchoring portion (2), and wherein the supporting bodies (24) preferably have further cross-bores (21).

9. The tibia nail according to any one of claims 1 to 8 wherein the first anchoring portion (2) has constricted areas between the cross-bores (12, 13).

10. The tibia nail according to any one of claims 1 to 9 wherein said nail has a chamfer (36) on one side at the free end of the second anchoring portion (5).

11. The tibia nail according to any one of claims 1 to 10 wherein the area moment of inertia about an axis perpendicular to the first axis is smaller by a factor of at least 2 than the one about an axis perpendicular to the second plane across at least one region of the flexurally resilient portion (3).

## Revendications

1. Clou tibial, comprenant une première partie d'ancrage (2) présentant au moins un alésage transversal (12, 13) destiné à l'ancrage à l'aide d'une vis de serrage (6), une tige (4) faisant suite à la première partie d'ancrage (2), et une deuxième partie d'ancrage (5), qui fait suite à la tige (4) en formant un angle (α) par rapport à cette dernière, et qui présente au moins un autre alésage transversal (31, 32), **caractérisé en ce que**, en tant que clou tibial rétrograde, il présente, entre la première partie d'ancrage (2) et la tige (4), une partie de jonction (3) élastiquement flexible et dont l'élasticité en flexion est plus grande dans le premier plan contenant les axes (9, 30) de la tige (4) et de la deuxième partie d'ancrage (5), que dans un deuxième plan qui s'étend perpendiculairement au premier plan et qui contient l'axe (9) de la tige (4).

2. Clou tibial selon la revendication 1, présentant un alésage longitudinal (7) traversant.

3. Clou tibial selon la revendication 1 ou 2, présentant, au niveau de l'extrémité libre de la première partie d'ancrage (2) et sur le côté, qui est diamétralement opposé à la deuxième partie d'ancrage (5) par rapport à l'axe (7) de la première partie d'ancrage (2), une partie fraisée (15) à la manière d'un patin.

4. Clou tibial selon l'une des revendications 1 à 3, dans lequel la première partie d'ancrage (2) présente plusieurs alésages transversaux (12, 13) selon une orientation différente, et dans lequel au moins l'un des alésages transversaux (12) s'étend perpendiculairement au premier plan.

5. Clou tibial selon l'une des revendications 1 à 4, dans lequel la deuxième partie d'ancrage (5) présente un trou oblong (32) ainsi qu'un alésage longitudinal taraudé (34) destiné à la mise en place d'une vis de compression (35), tandis que, de préférence, la deuxième partie d'ancrage (5) présente, en outre, un alésage transversal (31) cylindrique.

6. Clou tibial selon l'une des revendications 1 à 5, dans lequel l'angle (α) est compris entre 5° et 30°, avec, de préférence, une valeur d'environ 15°.

7. Clou tibial selon l'une des revendications 1 à 6, dans lequel la partie de jonction (3) présente des parties fraisées (22, 23), qui sont parallèles au deuxième plan et dans lequel, de préférence, les parties fraisées (23) sont plus profondes à partir d'un côté, notamment le côté antérieur, qu'à partir de l'autre côté, et les parties fraisées (23) les plus profondes coupant l'alésage longitudinal (7).

8. Clou tibial selon l'une des revendications 1 à 7, dans lequel la partie de jonction (3) est divisée par des corps d'appui (24), qui présentent approximativement le même diamètre extérieur que la première partie d'ancrage (2), et les corps d'appui (24) comportant, de préférence, des alésages transversaux (21) supplémentaires.

9. Clou tibial selon l'une des revendications 1 à 8, dans lequel la première partie d'ancrage (2) présente, entre les alésages transversaux (12, 13), des rétrécissements.

10. Clou tibial selon l'une des revendications 1 à 9, présentant, au niveau de l'extrémité libre de la deuxième partie d'ancrage (5), un chanfrein (36) d'un seul côté.

11. Clou tibial selon l'une des revendications 1 à 10, dans lequel, par l'intermédiaire d'au moins une zone de la partie élastiquement flexible (3), le moment d'inertie de surface, autour d'un axe perpendiculaire au premier plan, est plus petit d'au moins un facteur de 2 que ce n'est le cas autour d'un axe perpendiculaire au deuxième plan.
